(19) Europäisches Patentamt / European Patent Office / Office européen des brevets

(11) **EP 4 632 750 A1**

(12) **EUROPEAN PATENT APPLICATION**

(43) Date of publication:
**15.10.2025   Bulletin 2025/42**

(21) Application number: **25168420.5**

(22) Date of filing: **04.04.2025**

(51) International Patent Classification (IPC):
**G16C 20/30** $^{(2019.01)}$       **G16C 60/00** $^{(2019.01)}$

(52) Cooperative Patent Classification (CPC):
**G16C 20/30; G16C 60/00;** G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **10.04.2024   DE 102024110051**

(71) Applicant: **Bundesdruckerei GmbH**
**10969 Berlin (DE)**

(72) Inventors:
• **Kowalski, Hagen-Henrik**
**12307 Berlin (DE)**
• **Eble, Holger**
**10965 Berlin (DE)**
• **Simon, Lars**
**10117 Berlin (DE)**
• **Radons, Manuel**
**15712 Königs Wusterhausen (DE)**
• **Muth, Oliver**
**12277 Berlin (DE)**

(74) Representative: **Richardt Patentanwälte PartG mbB**
**Wilhelmstraße 7**
**65185 Wiesbaden (DE)**

(54) **TRAINING DATA FOR MATERIAL SOLUBILITY PREDICTION**

(57)     Disclosed is a method for generating a training dataset to be used in training a machine learning model in order to predict the solubility of a material in at least one solvent, the method comprising: performing the following repeatedly: entering a selection of hypothetical materials (solute and solvent) from a parameter space, the parameter space being defined by a set of material parameters describing the said hypothetical materials; using a thermostat to set a specific temperature value T of the solute-solvent system; using a classical computer to apply a path integral molecular dynamics (PIMD) technique to calculate a free energy change between a first state of the solute-solvent system and a second state of the solute-solvent system, wherein the forces acting on the nuclei of the solute-solvent system which are used by PIMD are determined by solving numerically, on a quantum computer, the electronic Schrödinger equation of a quantum system representing the solute-solvent system; calculating the solubility of the solute in the solvent at the specific temperature value T; adding an entry to the training dataset, the entry indicates the selected solute and the selected solvent and a label, wherein the label indicates the calculated solubility.

FIG. 2

## Description

## FIELD OF THE INVENTION

The disclosure relates to a method for generating a training dataset to be used in training a machine learning model to predict the solubility of a material in a solvent.

## BACKGROUND

The solubility prediction of a material in a solvent is based on resource-intensive calculations of dynamics of nuclei of constituent atoms of the material and the solvent using ab-initio or classical molecular dynamics (MD). These calculations are approximate, even on high-performance computers, since no efficient algorithm is known for the exact solution of the underlying mathematical optimization problem. In addition, the quantum nature of the nuclei is not considered in the ab-initio or the classical MD.

## SUMMARY

Example embodiments provide a method for generating a training dataset to be used in training a machine learning model to predict a solubility of a material in at least one solvent, the method comprising: performing the following repeatedly: entering a selection of a hypothetical material and another hypothetical material as a solvent from a parameter space, the parameter space being defined by a set of material parameters describing materials, an amount of the hypothetical material dissolved in the solvent defines a solute, the solute and the solvent defines a solute-solvent system; using a thermostat in order to set a specific temperature value T of the solute-solvent system; using a classical computer to apply a path integral molecular dynamics (PIMD) technique to calculate a free energy change between a first state of the solute-solvent system and a second state of the solute-solvent system, wherein the forces acting on the nuclei of the solute-solvent system which are used by PIMD are determined by solving numerically, on a quantum computer, the electronic Schrödinger equation of a quantum system representing the solute-solvent system; calculating the solubility of the hypothetical material in the solvent at the specific temperature value T; adding an entry to the training dataset, the entry indicates the selected hypothetical material and the selected solvent and a label, wherein the label indicates the calculated solubility.

Example embodiments provide a computer program comprising machine executable instructions, wherein execution of the machine executable instructions causes a computer system to perform at least the following: performing the following repeatedly: entering a selection of a hypothetical material and another hypothetical material as a solvent from a parameter space, the parameter space being defined by a set of material parameters describing materials, an amount of the hypothetical material dissolved in the solvent defines a solute, the solute and the solvent defines a solute-solvent system; using a thermostat in order to set a specific temperature value T of the solute-solvent system; using a classical computer to apply a path integral molecular dynamics (PIMD) technique to calculate a free energy change between a first state of the solute-solvent system and a second state of the solute-solvent system, wherein the forces acting on the nuclei of the solute-solvent system which are used by PIMD are determined by solving numerically, on a quantum computer, the electronic Schrödinger equation of a quantum system representing the solute-solvent system; calculating the solubility of the hypothetical material in the solvent at the specific temperature value T; adding an entry to the training dataset, the entry indicates the selected hypothetical material and the selected solvent and a label, wherein the label indicates the calculated solubility.

Example embodiments provide a computer system for generating a training dataset for training a machine learning model to predict the solubility of a material in a solvent, the computer system being configured for: performing the following repeatedly: entering a selection of a hypothetical material and another hypothetical material as a solvent from a parameter space, the parameter space being defined by a set of material parameters describing materials, an amount of the hypothetical material dissolved in the solvent defines a solute, the solute and the solvent defines a solute-solvent system; using a thermostat in order to set a specific temperature value T of the solute-solvent system; using a classical computer to apply a path integral molecular dynamics (PIMD) technique to calculate a free energy change between a first state of the solute-solvent system and a second state of the solute-solvent system, wherein the forces acting on the nuclei of the solute-solvent system which are used by PIMD are determined by solving numerically, on a quantum computer, the electronic Schrödinger equation of a quantum system representing the solute-solvent system; calculating the solubility of the hypothetical material in the solvent at the specific temperature value T; adding an entry to the training dataset, the entry indicates the selected hypothetical material and the selected solvent and a label, wherein the label indicates the calculated solubility.

## BRIEF DESCRIPTION OF THE DRAWINGS

In the following, examples are described in greater detail referring to the drawings in which:

Fig. 1 is a diagram illustrating a computer system in accordance with an example of the present subject matter.

Fig. 2 is a flowchart of a method for generating a training dataset to be used in training a machine learning model to predict a solubility of a material in accordance with an example of the present subject matter.

Fig. 3 is a diagram of a data structure representing the training dataset generated in accordance with an example of the present subject matter.

Fig. 4 is a diagram of a data structure representing the training dataset generated in accordance with an example of the present subject matter.

Fig. 5 is a diagram of a data structure representing the training dataset generated in accordance with an example of the present subject matter.

Fig. 6 is a flowchart of a method for training a classical machine learning model in accordance with an example of the present subject matter.

Fig. 7 is a flowchart of using a trained machine learning model for solubility prediction of a solute in a solvent in accordance with an example of the present subject matter.

Fig. 8 is a diagram illustrating different phases for enabling solubility prediction of materials in accordance with an example of the present subject matter.

Fig. 9 is a block diagram of an exemplary computer system for implementing at least part of the present method in accordance with an example of the present subject matter.

## DETAILED DESCRIPTION

In the following description, for purposes of explanation and not limitation, specific details are set forth such as particular architectures, interfaces, techniques, etc., to provide a thorough understanding of the examples. However, it will be apparent to those skilled in the art that the disclosed subject matter may be practiced in other illustrative examples that depart from these specific details. In some instances, detailed descriptions of well-known devices and/or methods are omitted so as not to obscure the description with unnecessary detail.

Identity (ID) document may need to be secured against counterfeiting. For that, different security features may be used in the ID document. The security features may include holograms, special inks, watermarks, security threads or other visual and physical elements. The present subject matter may enable the development of new polymeric ink-composition to be used for security features in ID documents by accurately predicting specific properties of the polymeric ink-composition, wherein the said property may be the solubility of the polymeric ink-composition in organic or aqueous medium at a specific temperature value. The durability, the effectiveness, and the longevity of the security features embedded in ID documents may be determined using the solubility of the polymeric ink-composition. In another example, it may be determined, based on the solubility of the polymeric ink-composition, whether the polymeric ink-composition may be applied on a color-based security element in an ID document. The color-based security element may require a specific solubility of the polymeric ink-composition for precise and uniform application. For example, machine learning models may be used to predict the solubility of materials. The present subject matter may provide optimal training data for training these models. The resulting trained models may have a higher accuracy of solubility prediction.

A training dataset may be generated to be used in training a machine learning model to predict a solubility of a material in at least one solvent at a specific temperature value T.

The training dataset may be created by a set of hypothetical materials. Entering a selection of a hypothetical material may refer to the act of choosing a hypothetical material as an entry in the training dataset. The hypothetical material which is provided as a solute may be referred to as a solute hypothetical material. The solute hypothetical material may be a material. The solute hypothetical material may be a material that could exist, but not have been made and/or characterized experimentally. The solute hypothetical material may, for example, be a solid. The solute hypothetical material may, for example, be a polymer. The polymer may be any natural or synthetic material e.g., composed of large molecules.

The hypothetical material which is provided as a solvent may be referred to as a solvent hypothetical material. The solvent hypothetical material may be a material. The solvent hypothetical material may be a material that could exist, but not have been made and/or characterized experimentally. The solvent hypothetical material may, for example, be a liquid. The solvent hypothetical material may, for example, be an aqueous medium. The solvent hypothetical material may, for example, be an organic medium.

Each solute hypothetical material and each solvent hypothetical material of the set of hypothetical materials may be selected from a parameter space. The parameter space may comprise solute hypothetical materials and solvent hypothetical materials that can be selected, wherein each solute hypothetical material and each solvent hypothetical material may have a hypothetical chemical composition and hypothetical electron configurations. In one example, the solute hypothetical material and the solvent hypothetical material may be selected such that the corresponding electronic Schrödinger equation of the quantum system representing the solute-solvent system may be solved numerically on a quantum computer and the PIMD performed on a classical computer. In one example, the selected materials may have a number of atoms smaller than a threshold. The quantum computer may utilize the principles of quantum mechanics to perform quantum computations by operating qubits.

The parameter space may be defined by a set of material parameters describing the solute hypothetical materials and the solvent hypothetical materials. The set of material parameters of the solute hypothetical material may provide a representation of the solute hypothetical material and capture precise details of the chemical composition and electron configurations within the solute hypothetical material. The set of material parameters of the solvent hypothetical material may provide a representation of the solvent hypothetical material and capture precise details of the chemical composition and electron configurations within the solvent hypothetical material.

The set of material parameters of the solute hypothetical material may, for example, describe an atomic structure of the solute hypothetical material. The set of material parameters of the solvent hypothetical material may, for example, describe an atomic structure of the solvent hypothetical material. The material parameter may, for example, be anyone of: nuclear charge, electron configuration, orbital radius, electronegativity, and ionisation energy.

The parameter space may thus propose a solute hypothetical material and a solvent hypothetical material enabling new useful functional materials. Each selected solute hypothetical material may represent a point in the parameter space having specific values of the set of material parameters which are allowed by the parameter space. For example, the points in the parameter space may be defined using existing solute hypothetical materials. The parameter space may enable the design of new solute hypothetical materials. Each selected solvent hypothetical material may represent a point in the parameter space having specific values of the set of material parameters which are allowed by the parameter space. For example, the points in the parameter space may be defined using existing solvent hypothetical materials. The parameter space may enable the design of new solvent hypothetical materials.

The solubility may be defined as the ability of the solute hypothetical material to form a solution with the solvent hypothetical material. The solubility may be defined as the maximum amount of the solute hypothetical material that is dissolved in a specific amount of the solvent hypothetical material to form a stable solution at a specific temperature and pressure. The stable solution may be a solution in which no more of the solute hypothetical material can dissolve at a specific temperature and pressure without forming a precipitate. The stable solution may be referred to as a solution at thermodynamic equilibrium. The solubility may depend on the composition of the solute hypothetical material and the composition of the solvent hypothetical material, as well as on temperature and pressure, and may be expressed as a mass of the dissolved solute hypothetical material per a volume of solvent hypothetical material (gram/litre). The solubility may be defined by a solute mole fraction at thermodynamic equilibrium. The solute mole fraction may refer to moles of solute hypothetical material per total moles of solute hypothetical material plus solvent hypothetical material.

The selected solute hypothetical material and the selected solvent hypothetical material define a solute-solvent system. The solute-solvent system may define a quantum system. The quantum system may be defined for each selected solute hypothetical material and each selected solvent hypothetical material. This may result in a set of quantum systems. The quantum system may be described by a wavefunction. The wavefunction may constitute the most complete description of the quantum system. The Born-Oppenheimer approximation may enable separating the wavefunction of the quantum system into electronic part and nuclear part. The Born-Oppenheimer approximation may enable separating the time-independent Schrödinger equation of the quantum system into the electronic Schrödinger equation and the nuclear Schrödinger equation. According to the Born-Oppenheimer approximation, the electronic wavefunction and the electronic energy which are the solutions of the electronic Schrödinger equation may depend parametrically on nuclear positions. The position of the nucleus may be determined with respect to a reference point. The reference point may be an origin of a coordinate system.

A thermostat may refer to an algorithm to be used in a molecular dynamics (MD) simulation to maintain the temperature of a statistical system of molecules at a constant temperature value throughout the MD simulation. The use of the thermostat may comprise the execution of the thermostat algorithm. The thermostat algorithm in MD simulation may dynamically adjust particle velocities to maintain a constant temperature within the simulated system. The MD simulation may describe the time evolution of a system of atoms. A thermostat may be used to simulate a canonical ensemble, wherein the number of particles (atoms, molecules, etc.) in the solute-solvent system, the volume of the solute-solvent system, and the temperature of the solute-solvent system are kept constant. The canonical ensemble may be referred to it as NVT. The thermostat may, for example, be anyone of: Andersen thermostat, Langevin thermostat, Nosé-Hoover thermostat, and Berendsen thermostat. The free energy which is used in NVT may, for example, be the Helmholtz free energy.

The nuclear Schrödinger equation may depend parametrically on the solution of the electronic Schrödinger equation. The electronic energy obtained from the electronic Schrödinger equation may contribute to the potential energy of the nuclear Schrödinger equation. The said potential energy may define a potential energy surface. The forces acting on the nuclei may be obtained from the potential energy surface. The force acting on a nucleus may be obtained by the negative gradient of the potential energy surface at the position of the nucleus. The nuclear Schrödinger equation for each quantum system representing the respective solute hypothetical material and solvent hypothetical material may be solved numerically.

The quantum computer may be used for numerically solving the electronic Schrödinger equation for each quantum system representing the respective solute hypothetical material and the respective solvent hypothetical material. The electronic Schrödinger equation may, for example, be solved on the quantum computer using Full Configuration Interaction. The electronic Schrödinger equation may, for example, be solved on the quantum computer using contracted quantum eigensolver (CQE). The quantum computer may be a machine that uses the properties of quantum physics to store data and perform computations.

The time evolution of the average value of an observable may be obtained using the path integral molecular dynamics (PIMD). The observable may be provided for observing or measuring a specific property of the quantum system. The observable may be represented by an operator. The observable may, for example, be the energy. The classical computer may be used to apply the path integral molecular dynamics (PIMD) technique. The path integral molecular dynamics (PIMD) may comprise the path integral formalism and equations of motion. The equations of motion may, for example, be the Hamilton's equations of motion. The path integral formalism may be used to obtain the average value of the observable. The Hamilton's equations of motion may be used to calculate the updated quantum system after a time step. The path integral molecular dynamics may be used to include the nuclear quantum effects in the MD simulation.

According to the PIMD, A system of nuclei may be treated quantum mechanically by mapping each quantum nucleus onto a classical system of several fictitious particles, known as beads, connected by springs governed by an effective Hamiltonian which is derived from the path integral formalism. The classical system may be represented as a ring polymer. The beads in the ring polymer may have an index value. The set of beads which are specified by a particular index value may correspond to a classical analogue of the system of nuclei. Each of these sets of beads may be called a classical replica of the system of nuclei, or a replica.

A statistical mechanics partition function, denoted as Q, may be obtained from the path integral formalism. The partition function may represent a phase of the solute-solvent system.

The free energy change, at a specific temperature value T, between two states of the solute-solvent system represented by two partition functions Q2 and Q1 respectively, may be calculated using:

$$\Delta F = -k_B T ln(\frac{Q_2}{Q_1})$$

where $k_B$ is the Boltzmann constant, $ln$ represents the natural logarithm. The calculation may, for example, be carried out at an ambient pressure value. The first state may, for example, be a state when the solute hypothetical material is in its undissolved form in the solute-solvent system. The second state may, for example, be a state when the solute hypothetical material is in dissolved form in the solute-solvent system. The second state may, for example, be a state when the thermodynamic equilibrium is achieved in the solute-solvent system. The partition function may be for a canonical ensemble (NVT). The partition function may be for an isothermal-isobaric ensemble (NPT). The solubility may be calculated when the free energy change $\Delta F$ of the solute-solvent system equals zero. The calculation may, for example, be carried out at an ambient pressure value. This is, for example, described in the paper: Zoran Bjelobrk et al. Cryst. Growth Des. 2021, 21, 9, 5198 - 5205. At this condition, $\Delta F = 0$, the solution may be stable, and the solubility may correspond to the solute mole fraction. In one example, in order to determine the solubility, the PIMD may use the explicit solvent model. The solvent hypothetical material may be represented using the explicit solvent model. The explicit solvent model may explicitly include individual solute hypothetical material atoms and solvent hypothetical material atoms within the MD simulation.

In one example, in order to determine the solubility, the PIMD may use the implicit solvent model. The solvent hypothetical material may be represented using the implicit solvent model. The implicit solvent model may account for the effects of the solvent hypothetical material on the solute hypothetical material. For that, the equations that describe the behavior of the solute hypothetical material may include the effect of the solvent hypothetical material which approximates the global behavior of the solvent hypothetical material without explicitly considering the individual atoms of the solvent hypothetical material. In particular, the solvent hypothetical material effects may be incorporated into the Hamiltonian of the solute-solvent system. The implicit solvent model may estimate the solute-solvent interactions.

For each solute hypothetical material of the set of hypothetical materials, and for each solvent hypothetical material of the set of hypothetical materials, an entry may be included in the training dataset. The entry may comprise a description of the solute hypothetical material and a description of the solvent hypothetical material, and a label indicating the obtained solubility of the solute hypothetical material in the solvent hypothetical material. The description of the solute hypothetical material may, for example, comprise values of the set of material parameters. The description of the solvent hypothetical material may, for example, comprise values of the set of material parameters. Each entry, for example, of the training dataset may comprise a tuple (*Solute_i, Solvent_i, Label_i*), where *Solute_i* is the description of the i-th solute hypothetical material and *Solvent_i* is the description of the i-th solvent hypothetical material of the set of hypothetical materials and the *Label_i* is the label of the i-th solute hypothetical material and the i-th solvent hypothetical material. The training dataset may, for example, be stored in a storage system of the computer system. The computer system may control access to the training dataset. The computer system may, for example, define permissions, such as read permission and execute permissions, for access to the training dataset. One or more users may use the training dataset based on permissions which are assigned to the users.

According to one example, the at least one solvent is one solvent or a mixture of solvents. Each solvent hypothetical material may be selected from the parameter space. The mixture of the solvent hypothetical materials may change the solubility of the solute hypothetical material in comparison to the solubility of the solute hypothetical material in one solvent hypothetical material. This property may, for example, enhance the solubility of the solute hypothetical material to be used for a specific application.

According to one example, the specific temperature value is a temperature value in the range of -10 to 150 °C, preferably 0 to 100 °C, more preferably 5 to 30 °C, most preferably 15 to 30 °C, where °C refers to degree celsius. This example may be advantageous because the determination of the solubility of the solute hypothetical material in the solvent hypothetical material at the said temperature ranges may be relevant to real-world conditions. For example, the determination of the solubility at the room temperature range 15 to 30 °C may be advantageous because the solvent hypothetical material does not need to be heated up or cooled down before intended use. This may enable a most practical, a most economic and an easiest process to handle solutions that do not need to be heated up or cooled down before intended use. This may lead to an overall more efficient process.

According to one example, two or more specific temperature values are used to create the training dataset, wherein each entry of the training dataset further indicates the associated temperature value. This example may be useful because the solubility of the solute hypothetical material in water may, for example, be different when water is at room temperature and when water is at boiling temperature.

According to one example, a barostat may be used to set a specific pressure value P of the solute-solvent system. The barostat may refer to an algorithm to be used in a molecular dynamics (MD) simulation to maintain the pressure of a statistical system of molecules at a constant pressure value throughout the MD simulation. A barostat algorithm in MD simulation dynamically adjusts the volume of the simulation box to maintain a constant pressure within the simulated system. This is achieved by scaling the coordinates of atoms in the simulation box. The use of the barostat may comprise the execution of the barostat algorithm. The MD simulation may describe the time evolution of a system of atoms. A barostat may be used with a thermostat to simulate an isothermal-isobaric ensemble, wherein the number of particles (atoms, molecules, etc.) in the solute-solvent system, the pressure of the solute-solvent system, and the temperature of the solute-solvent system are kept constant. The isothermal-isobaric ensemble may be referred to it as NPT. The barostat may, for example, be anyone of: Berendsen barostat, Andersen barostat, Parrinello-Rahman barostat. The free energy which is used in NPT may, for example, be the Gibbs free energy. The calculations in the above examples may be carried out at an ambient pressure value.

According to one example, the two or more specific pressure values are used to create the training dataset, wherein each entry of the training dataset further indicates the associated pressure value.

According to one example, the parameter space comprises points, each point has the corresponding values of the set of material parameters, wherein the parameter space is defined using a bin packing problem such that the parameter space is uniformly filled by the points. The bin packing problem may enable an optimization problem, in which items of different sizes may be packed into a finite number of bins or containers, each of a fixed given capacity, in a way that minimizes the number of bins used. This example may enable controlling the size as well as the structure of the parameter space.

According to one example, the parameter space is defined by a Smooth Overlap of Atomic Positions (SOAP) descriptor. The SOAP descriptor may characterize the solute hypothetical material and the solvent hypothetical material. The descriptor of the said materials may, for example, be a tuple of real valued functions of the atomic positions, e.g., bond lengths, bond angles, etc. of the said materials. The SOAP descriptors may provide appropriate representation of the said materials structures. This may further improve the accuracy of the predictions of the trained model because the model is trained based on accurate representations of the solute hypothetical materials and the solvent hypothetical materials. Another advantage may be that the level of details of the descriptors may be chosen for enabling coarser or finer fingerprinting. Coarser fingerprinting may save resources while providing reliable results especially as the required accuracy for the solubility prediction is less critical. If the accuracy in solubility predictions is high, fingerprinting may be finer. The descriptor may, for example, be provided as a vector whose entries represent the set of material parameters respectively.

According to one example, the set of material parameters being descriptive of chemical composition and atomic configuration of the solute hypothetical material and the solvent hypothetical material, wherein the set of material parameters comprises: nuclear charge, electron configuration, orbital radius, electronegativity, and ionisation energy.

According to one example, the method further comprising using the training dataset to train the machine learning model to predict the solubility of a solute hypothetical material in a solvent hypothetical material at a specific temperature value. For example, based on the training dataset, a classical machine learning model is trained using as input the chemical composition, the structure as well as the atomic properties of the different elements of the solute hypothetical material and the solvent hypothetical material to predict the solubility of the solute hypothetical material in the solvent hypothetical material at temperature value T. By using accurately determined solubilities to train the machine learning model, the predictions may be similarly at high accuracy level. The model may make accurate predictions without the use of a quantum computer. By contrast to classical or ab-initio molecular dynamics-based calculations which does not take into account the quantum nature of nuclei may typically give less accurate predictions of the solubility, the present subject matter may provide more accurate predictions of the solubility.

The machine learning model may, for example, be neural network, or deep neural network such as convolutional neural network (CNN). The machine learning model may quickly make predictions about material solubility even on classical computers. The present approach may be a hybrid approach because it uses the quantum computer to solve numerically the electronic Schrödinger equation of the quantum system representing the solute-solvent system, and it generates training dataset and then uses the trained model on a classical computer. The precision achieved with the machine learning model may not be achieved with purely classical methods (i.e., without using a quantum computer).

According to one example, the present method comprises: using the solute hypothetical material as, for example, a coating for security features in an identity (ID) document.

According to one example, the method further comprises: predicting the solubility of a solute hypothetical material in a solvent hypothetical material using the trained machine learning model; the predicted solubility may fulfil a selection criterion to use the solute hypothetical material as a coating for security features in, for example, an identity (ID) document. The selection criterion may require that the solubility, in a temperature range, is in a specific range. This may enable to provide highly secure ID documents using the material having the selected solubility.

According to one example, a description of a desired solute hypothetical material and a desired solvent hypothetical material may be received. The description of the desired solute hypothetical material and the desired solvent hypothetical material may be input to the trained machine learning model. An output of the trained machine learning model may be received. The output indicates the solubility of the desired solute hypothetical material in the desired solvent hypothetical material.

According to one example, the method comprises determining whether the selected solute hypothetical material has a desired solubility in the selected solvent hypothetical material using the predicted solubility. In case the selected solute hypothetical material has the desired solubility, the description of the selected solute hypothetical material may be input into a robotic system for coating an ID document using the selected solute hypothetical material. The robotic system includes automated chemical reactors and a system for implementing material deposition techniques. The robotic system includes automated chemical reactors and material deposition techniques under precise control over temperature, pressure, and composition.

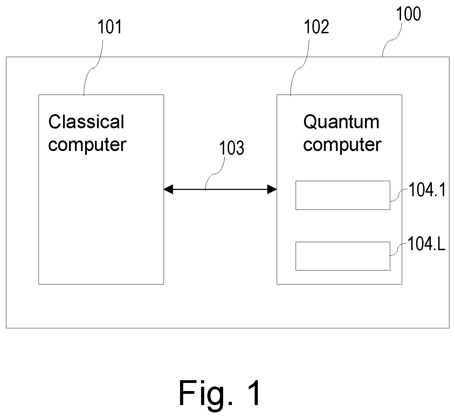

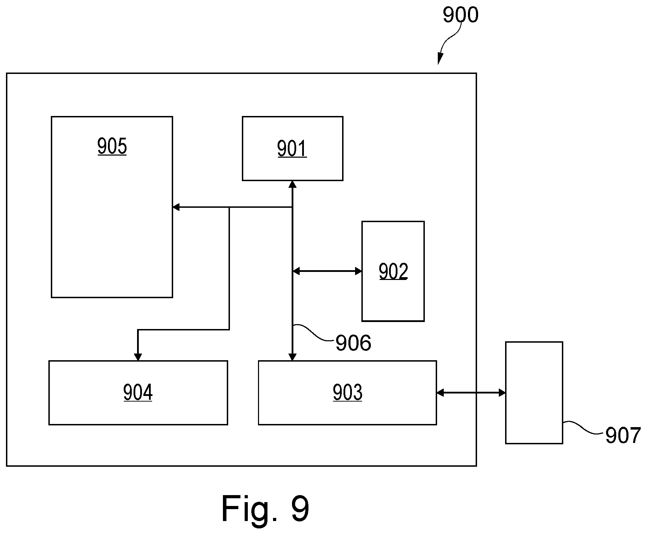

**Fig.1** is a diagram illustrating a computer system in accordance with an example of the present subject matter. The computer system 100 comprises a classical computer 101. The computer system 100 may further comprise a quantum computer 102. An example implementation of the classical computer 101 is described with reference to Fig. 9. The quantum computer 102 may comprise qubits. For example, qubits may be part of quantum registers 104.1 through 104.L. The classical computer 101 may be configured to control operation of the quantum computer 102. The classical computer 101 may use an interface 103 with the quantum computer 102 to control operation of the quantum computer 102 in accordance with an example of the present subject matter.

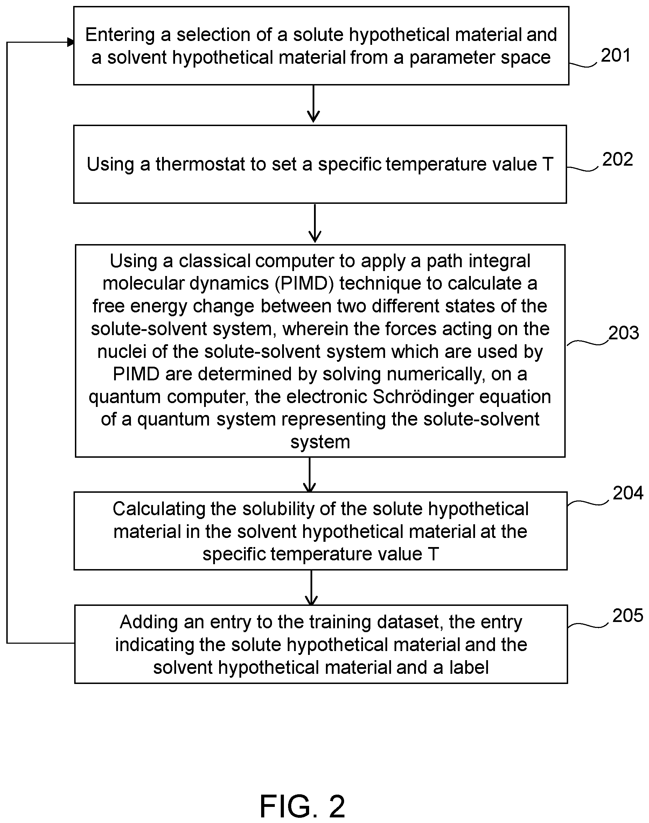

**Fig. 2** is a flowchart of a method for generating a training dataset to be used in training a machine learning model to predict a solubility of a material in accordance with an example of the present subject matter. For the purpose of explanation, the method described in Fig. 2 may be implemented in the system illustrated in Fig. 1 but is not limited to this implementation. For example, the method of Fig. 2 may be performed by the computer system 100.

A solute hypothetical material and a solvent hypothetical material may be entered as a selection in step 201 from a parameter space. The parameter space is defined by a set of material parameters describing the solute hypothetical materials and the solvent hypothetical materials.

A thermostat may be used in step 202 to set a specific temperature value T throughout the molecular dynamics (MD) simulation. A thermostat may refer to an algorithm. A thermostat algorithm in MD simulations may dynamically adjust particle velocities to maintain a constant temperature within the simulated system.

A classical computer (e.g., 101) may be used in step 203 to apply a path integral molecular dynamics (PIMD) technique to calculate a free energy change between two different states of the solute-solvent system, wherein the forces acting on the nuclei of the solute-solvent system which are used by PIMD are determined by solving numerically, on a quantum computer (e.g., 102), the electronic Schrödinger equation of a quantum system representing the solute-solvent system. The step 203 may, for example, be implemented following a procedure as follows: the Hamiltonian of the quantum system representing the solute-solvent system may be separated, on the classical computer, using the Born-Oppenheimer approximation, into the electronic part and the nuclear part. The electronic part of the Hamiltonian may be sent to the quantum computer where the electronic Schrödinger equation may be solved numerically, and the calculated electronic energy may be sent to the classical computer where the PIMD is executed. The partition function may be obtained from the path integral formalism and a free energy at a specific state of the solute-solvent system may be obtained from the partition function. The outcome of the PIMD may be the updated quantum system after a time step. The Hamiltonian of the updated quantum system may undergo the procedure as outlined above.

The solubility of the solute hypothetical material in the solvent hypothetical material at the specific temperature value T is calculated in step 204.

An entry may be added in step 205 to the training dataset. The entry indicates the selected solute hypothetical material and the selected solvent hypothetical material and a label, wherein the label indicates the obtained solubility at the specific temperature value T. The entry may comprise a description of the solute hypothetical material and the solvent hypothetical material and the label which is the solubility of the solute hypothetical material in the solvent hypothetical material.

As indicated in Fig. 2, steps 201 to 205 may be repeated for each further selected solute hypothetical material and solvent hypothetical material. The repetition may be performed until a stopping criterion is fulfilled. The stopping criterion may, for example, require that a maximum number of repetitions is reached or that a number of entries of the training dataset is reached.

In one example implementation of Fig. 2, the whole method may be repeated e.g., on a periodic basis, wherein the training dataset is updated/increased with new entries obtained for each further execution of the method.

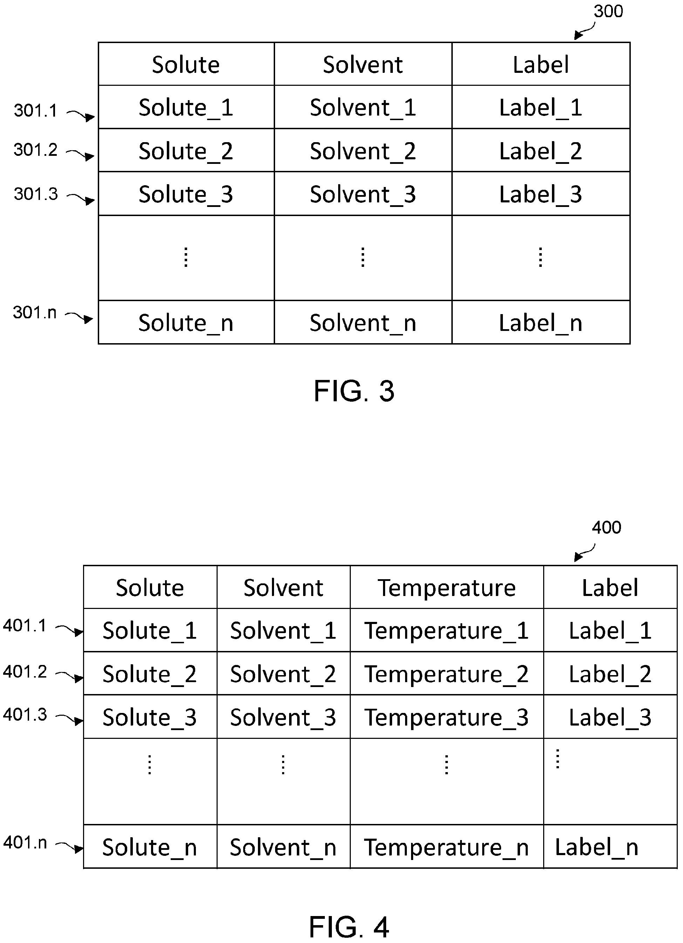

Fig. 3 is a diagram of a data structure representing the training dataset generated in accordance with an example of the present subject matter. The data structure 300 comprises n entries 301.1 through 301.n. Each i-th entry 301.i of the data structure comprises a tuple (*Solute_i, Solvent_i, Label_i*), where So*lute_i* is the description of the i-th solute hypothetical material and *Solvent_i* is the description of the i-th solvent hypothetical material of the set of hypothetical materials and the *Label_i* is the solubility of the i-th solute hypothetical material in the i-th solvent hypothetical material at the specific temperature T.

Fig. 4 is a diagram of a data structure representing the training dataset generated in accordance with an example of the present subject matter. The data structure 400 comprises n entries 401.1 through 401.n. Each i-th entry 401.i of the data structure comprises a tuple (*Solute_i, Solvent_i, Temperature_i, Label_i*), where *Solute_i* is the description of the i-th solute hypothetical material and *Solvent_i* is the description of the i-th solvent hypothetical material of the set of hypothetical materials and *Temperature_i* is the temperature value under which the solubility is calculated and the *Label_i* is the solubility of the i-th solute hypothetical material in the i-th solvent hypothetical material at a temperature value *Temperature_i*. Different i-th entries may have the same temperature value.

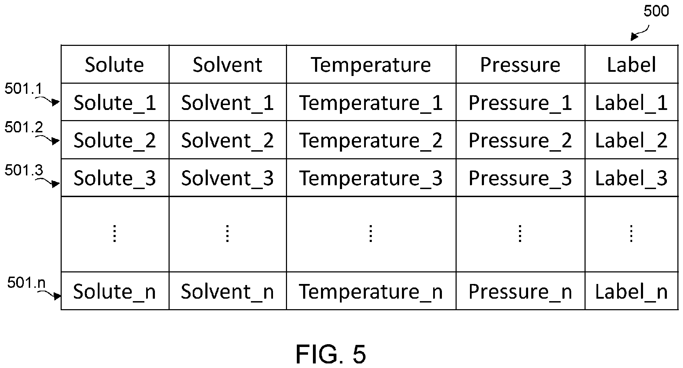

Fig. 5 is a diagram of a data structure representing the training dataset generated in accordance with an example of the present subject matter. The data structure 500 comprises n entries 501.1 through 501.n. Each i-th entry 501.i of the data structure comprises a tuple (*Solute_i, Solvent_i, Temperature_i, Pressure_i, Label_i*), where *Solute_i* is the description of the i-th solute hypothetical material and *Solvent_i* is the description of the i-th solvent hypothetical material of the set of hypothetical materials and *Temperature_i* is the temperature value under which the solubility is calculated and *Pressure_i* is the pressure value under which the solubility is calculated and the *Label_i* is the solubility of the i-th solute hypothetical material in the i-th solvent hypothetical material at a temperature value *Temperature_i* and a pressure value *Pressure_i*. Different i-th entries may have the same temperature value but different pressure values. Different i-th entries may have the same pressure value but different temperature values.

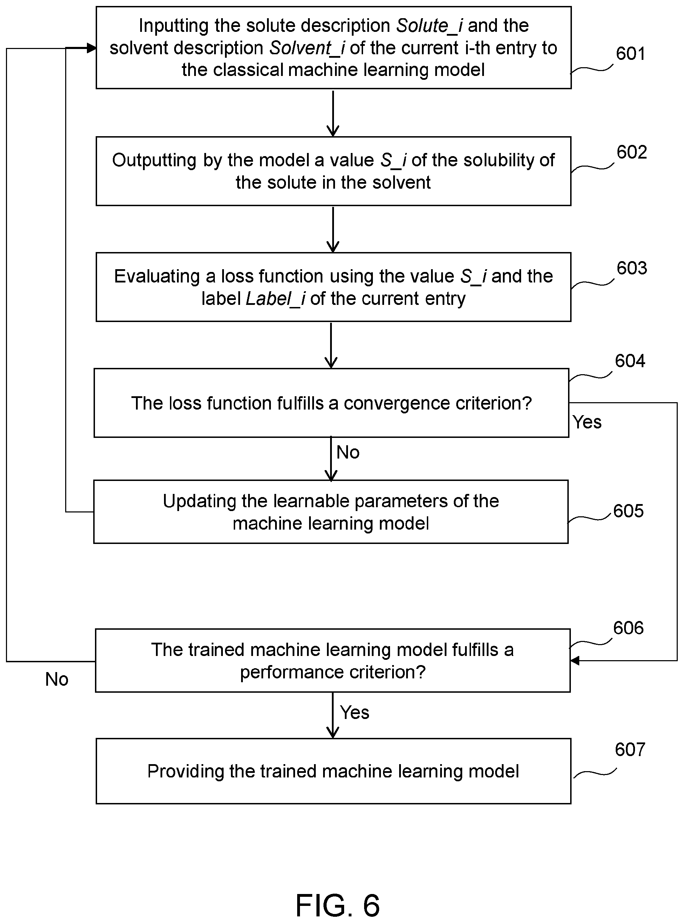

Fig. 6 is a flowchart of a method for training a classical machine learning model in accordance with an example of the present subject matter. The training may be performed using the training dataset as generated, for example, by the method of Fig. 2. For simplification, Fig. 6 may be described with reference to the data structure of Fig. 3. The entries 301.1 to 301.n may be processed one by one using the method. The classical machine learning model may, for example, be a deep neural network such as a CNN or a support vector machine.

In step 601, the description *Solute_i* and the description *Solvent_i* of the current i-th entry 301.i may be input to the classical machine learning model. In response, the machine learning model may ouput in step 602 a value *S_i* of the solubility of the solute in the solvent. A loss function may be evaluated in step 603 using the value *S_i* and the label *Label_i* of the current entry 301.i. In step 604, in case the loss function does not fulfill a convergence criterion, the learnable weights of the machine learning model may be updated in step 605 and steps 601 to 604 may be repeated for a next entry of the training dataset 300. The update of the learnable weights may be performed using gradient descent. For example, the convergence criterion may be defined using a numerical toelrance value. The convergence criterion may, for exmaple, be fulfilled if the difference of the loss function between two iterations is less than that tolerance value. If this tolerance value is not achieved, a warning may be provided and/or the training may be repeated. The training may, for example, be repeated with a larger tolerance value. For exmaple,the tolerance value may be changed if the training takes too long or if the results are not accurate enough e.g., the change may be performed by choosing a looser criterion that may lead to a shorter training time. In case the loss function fulfills the convergence criterion, it may be determined in step 606 whether the trained machine learning model fulfils a performance criterion. The performance criterion may be defined in accordance with a cross-validation. The cross-validation may be used to evaluate the performance of the trained model on a validation dataset. The validation dataset may, for example, comprise unseen data. The performance criterion may require the trained machine learning model surpasses predefined thresholds for one or more performance evaluation metrics. In case the trained machine learning model fulfils the performance criterion, the trained machine learning model may be provided in step 607. In case the trained machine learning model does not fulfil the performance criterion, the method may be repeated by going back to step 601 for retraining the machine learning model. The repetition may be performed using a different training dataset for training and a different validation dataset for the validation in step 606. The repetition may be performed until the trained machine learning model is validated. The validation may ensure that the model generalizes to data it has not been trained on. This may enable to avoid overfitting or underfitting.

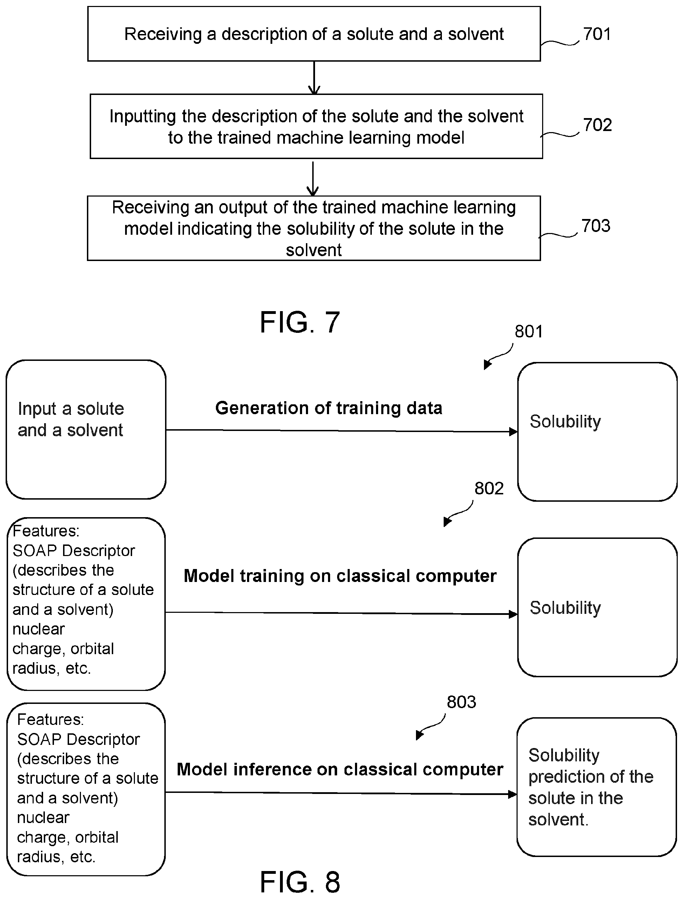

**Fig. 7** is a flowchart of using a trained machine learning model for solubility prediction of a solute in a solvent in accordance with an example of the present subject matter.

A description of the solute and a description of a solvent may be received in step 701. The said descriptions may be input in step 702 to a trained machine learning model (e.g., which is obtained in Fig. 6). An output of the trained machine learning model may be received in step 703 from the trained machine learning model. The output indicates the solubility of the input solute in the input solvent.

The method of Fig. 7 may, for example, be performed by the computer system of Fig. 9.

**Fig. 8** is a diagram illustrating different phases for enabling solubility prediction of materials in accordance with an example of the present subject matter. The phases include a first phase 801 for generation of training dataset. The first phase 801 uses as input a solute and a solvent in order to determine the solubility of the input solute in the input solvent. The phases include a second phase 802 for training a machine learnining model on a classical computer system using the training dataset obtained in the first phase 801. The machine learning model may use as input the description of the solute and the solvent of the training dataset in order to be trained to predict the solubility of the solute in the solvent. The description of the solute and the solvent may comprise features which are obtained by the SOAP descriptor. The phases include a third phase 803 for using or inference of the trained machine learning model which is obtained from the second phase 802. The trained machine learning model may receive as input the SOAP descriptor of the solute and the solvent and may provide as output a solubility prediction of the solute in the solvent.

**Fig. 9** is a block diagram of an exemplary computer system for implementing at least part of the present method in accordance with an example of the present subject matter.

The components of the computer system 900 may include, but are not limited to, one or more processors or processing units 901, a storage system 902, a memory unit 905, and a bus 906 that couples various system components including memory unit 905 to processor 901. The storage system 902 may include for example a hard disk drive (HDD). The memory unit 905 may include computer system readable media in the form of volatile memory, such as random access memory (RAM) and/or cache memory.

The computer system 900 may also communicate with one or more external devices such as a keyboard, a pointing device, a display 907, etc.; one or more devices that enable a user to interact with computer system 900; and/or any devices (e.g., network card, modem, etc.) that enable the computer system 900 to communicate with one or more other computing devices. Such communication can occur via I/O interface(s) 903. Still yet, the computer system 900 can communicate with one or more networks such as a local area network (LAN), a general wide area network (WAN), and/or a public network (e.g., the Internet) via a network adapter 904. As depicted, the network adapter 904 communicates with the other components of the computer system 900 via bus 906.

The memory unit 905 is configured to store applications that are executable on the processor 901. For example, the memory unit 905 may comprise an operating system as well as one or more application programs. The application programs comprise instructions that when executed enable to perform the method described with reference to Fig. 2, 6, or 7.

As will be appreciated by one skilled in the art, aspects of the present invention may be embodied as an apparatus, method, computer program or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

A computer program comprises the computer executable code or 'program instructions'.

The term 'computer system' refers to data processing hardware and encompasses all kinds of apparatus, devices, and machines for processing data, including by way of example, a programmable processor, a computer, or multiple processors or computers. The apparatus can also be or further include special purpose logic circuitry, e.g., a central processing unit (CPU), a FPGA (field programmable gate array), or an ASIC (application specific integrated circuit). In some implementations, the data processing apparatus and/or special purpose logic circuitry may be hardware-based and/or software-based. The apparatus can optionally include code that creates an execution environment for computer programs, e.g., code that constitutes processor firmware, a protocol stack, a database management system, an operating system, or a combination of one or more of them. The present disclosure contemplates the use of data processing apparatuses with or without conventional operating systems, for example LINUX, UNIX, WINDOWS, MAC OS, ANDROID, IOS or any other suitable conventional operating system.

Any combination of one or more computer readable medium(s) may be utilized. The computer readable medium may be a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the 'C' programming language or similar programming languages and compiled into machine executable instructions. In some instances the computer executable code may be in the form of a high level language or in a precompiled form and be used in conjunction with an interpreter which generates the machine executable instructions on the fly.

Generally, the program instructions can be executed on one processor or on several processors. In the case of multiple processors, they can be distributed over several different entities. Each processor could execute a portion of the instructions intended for that entity. Thus, when referring to a system or process involving multiple entities, the computer program or program instructions are understood to be adapted to be executed by a processor associated or related to the respective entity.

While the invention has been illustrated and described in detail in the drawings and foregoing description, such illustration and description are to be considered illustrative or exemplary and not restrictive; the invention is not limited to the disclosed examples.

**Claims**

1. A method for generating a training dataset to be used in training a machine learning model to predict a solubility of a material in at least one solvent, the method comprising: performing the following repeatedly:

   entering a selection of a hypothetical material and another hypothetical material as a solvent from a parameter space, the parameter space being defined by a set of material parameters describing materials, an amount of the hypothetical material dissolved in the solvent defines a solute, the solute and the solvent defines a solute-solvent system;
   using a thermostat in order to set a specific temperature value T of the solute-solvent system;
   using a classical computer to apply a path integral molecular dynamics (PIMD) technique to calculate a free energy change between a first state of the solute-solvent system and a second state of the solute-solvent system, wherein forces acting on a nuclei of the solute-solvent system which are used by PIMD are determined by solving numerically, on a quantum computer, the electronic Schrödinger equation of a quantum system representing the solute-solvent system;

calculating the solubility of the hypothetical material in the solvent at the specific temperature value T;

adding an entry to the training dataset, the entry indicates the selected hypothetical material and the selected solvent and a label, wherein the label indicates the calculated solubility.

2. The method of claim 1, wherein the at least one solvent is one solvent or a mixture of solvents.

3. The method of any of the preceding claims, wherein the specific temperature value is a temperature value in the range of -10 °C to 150 °C, preferably 0 °C to 100 °C, more preferably 5 °C to 30 °C, most preferably 15 °C to 30 °C .

4. The method of any of the preceding claims, wherein two or more specific temperature values are used to create the training dataset, wherein each entry of the training dataset further indicates an associated temperature value.

5. The method of any of the preceding claims, wherein a barostat is used to set a specific pressure value P of the solute-solvent system;

6. The method of any of the preceding claims, wherein two or more specific pressure values are used to create the training dataset, wherein each entry of the training dataset further indicates an associated pressure value.

7. The method of any of the preceding claims, wherein the parameter space comprises points, each point has the corresponding values of the set of material parameters, wherein the parameter space is defined using a bin packing problem such that the parameter space is uniformly filled by the points.

8. The method of any of the preceding claims, wherein the parameter space is defined by a Smooth Overlap of Atomic Positions (SOAP) descriptor.

9. The method of any of the preceding claims, the set of material parameters being descriptive of chemical composition and atomic configuration of the material, wherein the set of material parameters comprises: nuclear charge, electron configuration, orbital radius, electronegativity, and ionisation energy.

10. The method of any of the preceding claims, further comprising using the training dataset to train the machine learning model to predict the solubility of a hypothetical material in a solvent.

11. The method of any of the preceding claims, further comprising: using the selected hypothetical material as a coating for security features in an identity (ID) document.

12. A computer program comprising machine executable instructions, wherein execution of the machine executable instructions causes a computer system to perform at least the following: performing the following repeatedly:

entering a selection of a hypothetical material and another hypothetical material as a solvent from a parameter space, the parameter space being defined by a set of material parameters describing materials, an amount of the hypothetical material dissolved in the solvent defines a solute, the solute and the solvent defines a solute-solvent system;

using a thermostat in order to set a specific temperature value T of the solute-solvent system;

using a classical computer to apply a path integral molecular dynamics (PIMD) technique to calculate a free energy change between a first state of the solute-solvent system and a second state of the solute-solvent system, wherein forces acting on a nuclei of the solute-solvent system which are used by PIMD are determined by solving numerically, on a quantum computer, the electronic Schrödinger equation of a quantum system representing the solute-solvent system;

calculating the solubility of the hypothetical material in the solvent at the specific temperature value T;

adding an entry to a training dataset, the entry indicates the selected hypothetical material and the selected solvent and a label, wherein the label indicates the calculated solubility.

13. A computer system for generating a training dataset for training a machine learning model to predict a solubility of a material in a solvent, the computer system being configured for: performing the following repeatedly:

entering a selection of a hypothetical material and another hypothetical material as a solvent from a parameter space, the parameter space being defined by a set of material parameters describing materials, an amount of the hypothetical material dissolved in the solvent defines a solute, the solute and the solvent defines a solute-solvent system;

using a thermostat in order to set a specific temperature value T of the solute-solvent system;

using a classical computer to apply a path integral molecular dynamics (PIMD) technique to calculate a free energy change between a first state of the solute-solvent system and a second state of the solute-solvent system, wherein forces acting on a nuclei of the solute-solvent system which are used by PIMD are determined by solving numerically, on a quantum computer, the electronic Schrödinger equation of a quantum system representing the solute-solvent system;

calculating the solubility of the hypothetical material in the solvent at the specific temperature value T;

adding an entry to the training dataset, the entry indicates the selected hypothetical material and the selected solvent and a label, wherein the label indicates the calculated solubility.

14. A method for solubility prediction, the method comprising:

receiving a description of a selected material and a selected solvent;

inputting the description to a trained machine learning model, wherein the machine learning model is trained using training dataset of claim 1;

receiving an output of the trained machine learning model indicating the solubility of the selected material in the selected solvent.

15. The method of claim 14, further comprising,

determining whether the selected material has a desired solubility in the selected solvent using the predicted solubility;

in case the selected material has the desired solubility, inputting the description of the selected material into a robotic system, the robotic system including automated chemical reactors, and material deposition techniques under precise control over temperature, pressure, and composition.

16. A computer program comprising machine executable instructions, wherein execution of the machine executable instructions causes a computer system to perform at least the following:

receiving a description of a selected material and a selected solvent;

inputting the description to a trained machine learning model, wherein the machine learning model is trained using training dataset of claim 1;

receiving an output of the trained machine learning model indicating the solubility of the selected material in the selected solvent.

17. A computer system for solubility prediction, the computer system being configured for:

receiving a description of a selected material and a selected solvent;

inputting the description to a trained machine learning model, wherein the machine learning model is trained using training dataset of claim 1;

receiving an output of the trained machine learning model indicating the solubility of the selected material in the selected solvent.

101 102 100

Classical computer

103

Quantum computer

104.1

104.L

Fig. 1

Entering a selection of a solute hypothetical material and a solvent hypothetical material from a parameter space ⌇201

↓

Using a thermostat to set a specific temperature value T ⌇202

↓

Using a classical computer to apply a path integral molecular dynamics (PIMD) technique to calculate a free energy change between two different states of the solute-solvent system, wherein the forces acting on the nuclei of the solute-solvent system which are used by PIMD are determined by solving numerically, on a quantum computer, the electronic Schrödinger equation of a quantum system representing the solute-solvent system ⌇203

↓

Calculating the solubility of the solute hypothetical material in the solvent hypothetical material at the specific temperature value T ⌇204

↓

Adding an entry to the training dataset, the entry indicating the solute hypothetical material and the solvent hypothetical material and a label ⌇205

# FIG. 2

300

| | Solute | Solvent | Label |
|---|---|---|---|
| 301.1 | Solute_1 | Solvent_1 | Label_1 |
| 301.2 | Solute_2 | Solvent_2 | Label_2 |
| 301.3 | Solute_3 | Solvent_3 | Label_3 |
| | ⋮ | ⋮ | ⋮ |
| 301.n | Solute_n | Solvent_n | Label_n |

FIG. 3

400

| | Solute | Solvent | Temperature | Label |
|---|---|---|---|---|
| 401.1 | Solute_1 | Solvent_1 | Temperature_1 | Label_1 |
| 401.2 | Solute_2 | Solvent_2 | Temperature_2 | Label_2 |
| 401.3 | Solute_3 | Solvent_3 | Temperature_3 | Label_3 |
| | ⋮ | ⋮ | ⋮ | ⋮ |
| 401.n | Solute_n | Solvent_n | Temperature_n | Label_n |

FIG. 4

500

| Solute | Solvent | Temperature | Pressure | Label |
|---|---|---|---|---|
| Solute_1 | Solvent_1 | Temperature_1 | Pressure_1 | Label_1 |
| Solute_2 | Solvent_2 | Temperature_2 | Pressure_2 | Label_2 |
| Solute_3 | Solvent_3 | Temperature_3 | Pressure_3 | Label_3 |
| ⋮ | ⋮ | ⋮ | ⋮ | ⋮ |
| Solute_n | Solvent_n | Temperature_n | Pressure_n | Label_n |

501.1
501.2
501.3
501.n

## FIG. 5

Inputting the solute description *Solute_i* and the solvent description *Solvent_i* of the current i-th entry to the classical machine learning model — 601

Outputting by the model a value *S_i* of the solubility of the solute in the solvent — 602

Evaluating a loss function using the value *S_i* and the label *Label_i* of the current entry — 603

The loss function fulfills a convergence criterion? — 604

Yes

No

Updating the learnable parameters of the machine learning model — 605

The trained machine learning model fulfills a performance criterion? — 606

No

Yes

Providing the trained machine learning model — 607

FIG. 6

Receiving a description of a solute and a solvent ⌇701

↓

Inputting the description of the solute and the solvent to the trained machine learning model ⌇702

↓

Receiving an output of the trained machine learning model indicating the solubility of the solute in the solvent ⌇703

# FIG. 7

801

Input a solute and a solvent → **Generation of training data** → Solubility

802

Features:
SOAP Descriptor (describes the structure of a solute and a solvent) nuclear charge, orbital radius, etc. → **Model training on classical computer** → Solubility

803

Features:
SOAP Descriptor (describes the structure of a solute and a solvent) nuclear charge, orbital radius, etc. → **Model inference on classical computer** → Solubility prediction of the solute in the solvent.

# FIG. 8

Fig. 9

| | Europäisches Patentamt European Patent Office Office européen des brevets | **EUROPEAN SEARCH REPORT** | **Application Number** EP 25 16 8420 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JARMO HUUSKONEN ET AL: "Aqueous Solubility Prediction of Drugs Based on Molecular Topology and Neural Network Modeling", JOURNAL OF CHEMICAL INFORMATION AND COMPUTER SCIENCES., vol. 38, no. 3, 18 May 1998 (1998-05-18), pages 450-456, XP055718358, US ISSN: 0095-2338, DOI: 10.1021/ci970100x * abstract * * figure 3 * | 1-17 | INV. G16C20/30 G16C60/00 |
| Y | Loschen Christoph ET AL: "New Developments in Prediction of Solid-State Solubility and Cocrystallization Using COSMO-RS Theory" In: "Computational Pharmaceutical Solid State Chemistry", 6 April 2016 (2016-04-06), John Wiley & Sons, Inc., Hoboken, New Jersey, XP093301552, ISBN: 978-1-118-70068-6 pages 211-233, DOI: 10.1002/9781118700686.ch9, Retrieved from the Internet: URL:http://api.wiley.com/onlinelibrary/tdm/v1/articles/10.1002%2F9781118700686.ch9> * 9.1 INTRODUCTION 9.2 COSMO-RS 9.3 PREDICTION OF DRUG SOLUBILITY USING COSMO-RS 9.4 SOLUBILITY PREDICTION WITH MULTIPLE REFERENCE SOLVENTS * | 1-17 | |

TECHNICAL FIELDS SEARCHED (IPC)

G16C

-/--

The present search report has been drawn up for all claims

| Place of search | Date of completion of the search | Examiner |
|---|---|---|
| Munich | 4 August 2025 | Beligny, Samuel |

EPO FORM 1503 03.82 (P04C01)

Europäisches
Patentamt

European
Patent Office

Office européen
des brevets

**EUROPEAN SEARCH REPORT**

**Application Number**

EP 25 16 8420

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Anonymous: "COSMO-RS theory - COSMO-RS 2023.1 documentation", , 8 December 2023 (2023-12-08), pages 1-5, XP093301557, Retrieved from the Internet: URL:https://web.archive.org/web/2023120806 4324/https://www.scm.com/doc/COSMO-RS/COSM O-RS_theory.html * page 1 * ----- | | |
| A | US 2023/129485 A1 (INTERX INC [US]; PEREYASLAVETS LEONID [US] ET AL.) 27 April 2023 (2023-04-27) * paragraph [0153]; table 3 * ----- | 1-17 | |

TECHNICAL FIELDS
SEARCHED      (IPC)

The present search report has been drawn up for all claims

| Place of search | Date of completion of the search | Examiner |
|---|---|---|
| Munich | 4 August 2025 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 8420

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2025

| Patent document cited in search report | Publication date | Patent family member(s) | Publication date |
|---|---|---|---|
| US 2023129485 A1 | 27-04-2023 | EP 4445378 A1<br>US 2023129485 A1<br>WO 2023076956 A1 | 16-10-2024<br>27-04-2023<br>04-05-2023 |

EPO FORM P0459

For more details about this annex : see Official Journal of the European Patent Office, No. 12/82

**REFERENCES CITED IN THE DESCRIPTION**

**Non-patent literature cited in the description**

- **ZORAN BJELOBRK et al.** *Cryst. Growth Des.*, 2021, vol. 21 (9), 5198-5205